(19) Europäisches Patentamt
European Patent Office
Office européen des brevets

(11) EP 0 597 776 B1

# (12) FASCICULE DE BREVET EUROPEEN

(45) Date de publication et mention de la délivrance du brevet:
**14.01.1998 Bulletin 1998/03**

(51) Int Cl.[6]: **C07H 15/203**, A61K 7/48, A61K 7/16, A23L 1/05

(21) Numéro de dépôt: **93402758.2**

(22) Date de dépôt: **12.11.1993**

(54) **Monoesters de 4-hydroxy-phényl-Bêta-D-glucose, leur procédé de préparation et leurs utilisations dans les domaines cosmétique, pharmaceutique, bucco-dentaire et alimentaire**

4-Hydroxy-phenyl-beta-D-glukose-monoester, Verfahren zur ihrer Herstellung und ihre Verwendung in der Kosmetik, der Pharmazie, als Zahnpflegemitteln und Lebensmitteln

4-Hydroxy-phenyl-Beta-D-glucose monoesters, their method of preparation and their use in the cosmetic, dental-care, pharmaceuticals and food stuff fields

(84) Etats contractants désignés:
**AT BE CH DE ES FR GB GR IT LI NL PT SE**

(30) Priorité: **12.11.1992 FR 9213599**

(43) Date de publication de la demande:
**18.05.1994 Bulletin 1994/20**

(73) Titulaire: **L'OREAL**
**75008 Paris (FR)**

(72) Inventeur: **Philippe, Michel**
**F-91320 Wissous (FR)**

(74) Mandataire: **Stalla-Bourdillon, Bernard**
**CABINET NONY & CIE**
**29, rue Cambacérès**
**75008 Paris (FR)**

(56) Documents cités:
**EP-A- 0 117 413**
**EP-A- 0 319 993**
**US-A- 4 663 444**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 597 776 B1

## Description

La présente invention a pour objet de nouveaux monoesters du 4-hydroxy-phényl-β-D-glucose ou arbutine, leur procédé de préparation et leurs applications dans les domaines cosmétique, pharmaceutique, bucco-dentaire et alimentaire.

Les monoesters du 4-hydroxy-phényl-β-D-glucose sur l'hydroxyle du noyau phényle n'ont pas fait l'objet de recherches systématiques en vue de mettre en évidence certaines propriétés pouvant trouver une utilité dans différents domaines de l'industrie. Des recherches ont donc été conduites dans ce domaine et l'on a constaté de façon tout à fait surprenante que ces monoesters du 4-hydroxy-phényl-β-D-glucose peuvent être obtenus avec une bonne sélectivité et que ceux-ci présentaient des propriétés particulièrement intéressantes dans les domaines cosmétique, pharmaceutique, bucco-dentaire et alimentaire notamment du fait de leur propriété épaississante.

La présente invention a donc pour objet à titre de produits industriels nouveaux les monoesters du 4-hydroxy-phényl-β-D-glucose répondant à la formule générale suivante :

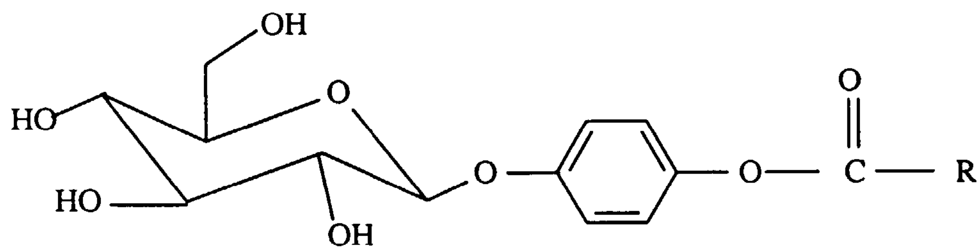
(I)

dans laquelle :
R représente un radical alkyle, linéaire ou ramifié ou alcényle ayant de 5 à 21 atomes de carbone ou un radical alcapolyényle ayant de 9 à 21 atomes de carbone ou R représente un mélange défini de tels radicaux alkyle, alcényle ou alcapolyényle.

Le radical alcapolyényle est de préférence un radical alcadiényle ou alcatriényle.

Parmi les monoesters du 4-hydroxy-phényl-β-D-glucose correspondant à la formule générale I on peut notamment citer :

4-hexanoyl-oxy-phényl-β-D-glucose,
4-décanoyl-oxy-phényl-β-D-glucose,
4-dodécanoyl-oxy-phényl-β-D-glucose,
4-hexadécanoyl-oxy-phényl-β-D-glucose,
4-oléoyl-oxy-phényl-β-D-glucose, et
4-linoléoyl-oxy-phényl-β-D-glucose.

La présente invention a également pour objet un procédé de préparation régiosélective des monoesters du 4-hydroxy-phényl-β-D-glucose de formule I, ce procédé consistant en un premier temps à former dans un solvant organique, un anhydride mixte de formule II :

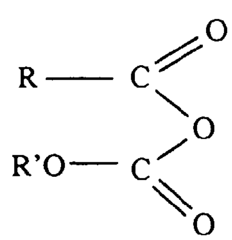
(II)

dans laquelle :
R représente un radical alkyle, linéaire ou ramifié ou alcényle, ayant de 5 à 21 atomes de carbone ou un radical alcapolyényle ayant de 9 à 21 atomes de carbone ou R représente un mélange défini de tels radicaux alkyle, alcényle ou alcapolyényle, et R' représente un radical alkyle, linéaire ou ramifié, ayant de 2 à 10 atomes de carbone, par réaction en présence d'une base, d'un acide R-COOH, R ayant la même signification que ci-dessus et d'un chloroformiate

d'alkyle ClCOOR', R' ayant la même signification que ci-dessus, et dans un deuxième temps, à faire réagir ledit anhydride mixte de formule II en solution dans ledit solvant organique ou dans un mélange de solvants organiques, avec du 4-hydroxy-phényl-β-D-glucose et éventuellement en présence d'une base.

Comme solvant organique réactionnel, on peut utiliser selon l'invention le tétrahydrofuranne, le N-N-diméthylformamide, la pyridine, la N-méthyl-pyrrolidone, le N,N-diméthylacétamide, le dichlorométhane ou un mélange de ces solvants.

La base servant à l'activation de l'acide est de préférence une base organique choisie parmi la triéthylamine, la pyridine, la 4-dimétylaminopyridine, la tributylamine ou encore la N-méthylmorpholine.

La deuxième étape ou étape d'estérification, peut être éventuellement réalisée après essorage des sels formés lors de la première étape et le 4-hydroxy-phényl-β-D-glucose est de préférence en solution dans la pyridine, ou éventuellement dans la N-méthylpyrrolidone, le N,N-diméthylformamide ou le N,N-diméthylacétamide. A cette solution de 4-hydroxy-phényl-β-D-glucose, on peut éventuellement ajouter une base choisie parmi la triéthylamine, la pyridine, la 4-diméthylaminopyridine, la tributylamine ou encore la N-méthylmorpholine.

On utilise de préférence, selon l'invention, au moins trois équivalents de 4-hydroxy-phényl-β-D-glucose par rapport à l'acide mis à réagir dans la première étape.

Selon l'invention, le chloroformiate d'alkyle est de préférence choisi parmi le chloroformiate d'éthyle et le chloroformiate d'isopropyle.

Après la fin de la réaction, les solvants sont évaporés et le produit résultant est extrait puis, soit chromatographié sur colonne de gel de silice, soit recristallisé dans un solvant organique approprié.

La température de réaction du procédé, selon l'invention, est généralement comprise entre -25 et 40°C le temps de réaction entre 6 et 15 heures.

L'acide RCOOH est de préférence l'acide hexanoïque, l'acide décanoïque, l'acide dodécanoïque, l'acide hexadécanoïque, l'acide oléique et l'acide linoléique.

La détermination des structures des produits obtenus a été réalisée par R.M.N$^{13}$C et R.M.N$^{1}$H dans le méthanol $D_4$ ou le D.M.S.O $D_6$.

Comme mentionné précédemment les monoesters du 4-hydroxy-phényl-β-D-glucose selon l'invention présentent de très intéressantes propriétés et trouvent donc des applications dans divers domaines de l'industrie et en particulier dans les domaines cosmétique, pharmaceutique, bucco-dentaire et alimentaire.

Parmi ces propriétés, celle qui a retenu tout particulièrement l'attention concerne la faculté qu'ont les monoesters du 4-hydroxy-phényl-β-D-glucose d'épaissir de nombreuses phases huileuses telles que notamment les triglycérides d'acides gras ayant de 8 à 12 atomes de carbone, le myristate d'isopropyle ou encore le palmitate de 2-éthyl-hexyle.

Ainsi on a constaté une prise en masse de triglycérides en $C_8$-$C_{12}$ ou du palmitate d'isopropyle par addition de 0,5 % en poids de 4-dodécanoyl-oxy-phényl-β-D-glucose (composé de formule I dans laquelle R = $C_{11}H_{23}$).

Le même effet a été constaté lorsque l'on utilise le 4-décanoyl-oxy-phényl-β-D-glucose (composé de formule I dans laquelle R = $C_9H_{19}$) lorsque celui-ci est employé à une concentration de 1% en poids.

On a également constaté pour les deux composés mentionnés ci-dessus que ceux-ci, à une concentration de 1% en poids, permettaient d'épaissir de façon très importante des solutions aqueuses.

Cette propriété épaississante des composés selon l'invention à l'égard d'une très large gamme de solvants était tout à fait inattendue et valorise leur intérêt dans de nombreux domaines de l'industrie.

On a également constaté que les composés selon l'invention peuvent s'intégrer très facilement à des membranes lipidiques pour former des vésicules permettant ainsi, dans le domaine de la cosmétique, une biodisponibilité facilitée au niveau de l'épiderme.

Les composés selon l'invention peuvent également être utilisés comme précurseurs de composés à activité dépigmentante.

La présente invention a également pour objet une composition cosmétique, pharmaceutique, bucco-dentaire ou alimentaire comprenant dans un véhicule approprié au moins un monoester du 4-hydroxy-phényl-β-D-glucose de formule I.

Dans les compositions selon l'invention le monoester du 4-hydroxy-phényl-β-D-glucose est généralement présent à une concentration comprise entre 0,01 et 30% en poids et de préférence entre 0,1 et 15% par rapport au poids total de la composition.

Les compositions selon l'invention peuvent se présenter sous différentes formes notamment sous forme de lotions moussantes ou non-moussantes, d'émulsions de consistance liquide ou semi-liquide telles que des laits obtenus par dispersion d'une phase grasse dans une phase aqueuse ou inversement, de suspensions ou d'émulsions de consistance molle de type crèmes ou pommades, de dispersions vésiculaires, de gels ou encore de préparations solides telles que des sticks, des pains de nettoyage, des tampons imprégnés ou encore sous forme de masques hydratants.
Comme véhicule des compositions selon l'invention on peut utiliser de l'eau, des solvants organiques compatibles avec une application topique tels que l'acétone, l'alcool isopropylique, l'alcool éthylique, les triglycérides d'acides gras en $C_6$-$C_{24}$, les éthers de glycol tels que les éthers d'alkyle inférieur de mono- ou dialkylène glycol dont le radical

alkylène a de deux à quatre atomes de carbone, les polyols comme le glycérol.

On peut également utiliser comme solvants des esters de polyalkylèneglycol et d'acide à chaîne courte en $C_1$-$C_4$ ou encore des silicones volatiles. Les compositions selon l'invention peuvent également contenir des corps gras tels que des huiles naturelles ou synthétiques et eventuellement d'autres épaississants différents de ceux de formule (I).

Les compositions selon l'invention peuvent également contenir des substances actives comme des agents hydratants ainsi que des adjuvants tels que des agents anti-oxydants, des agents conservateurs, des parfums, des colorants.

Les compositions selon l'invention peuvent également se présenter sous forme de solutions ou de dispersions contenant les monoesters du 4-hydroxy-phényl-β-D-glucose.

**Procédé général de préparation des monoesters du 4-hydroxy-phényl-β-D-glucose**

L'acide carboxylique R-COOH est mis en solution dans un solvant organique tel que le tétrahydrofuranne à 25% (P/V). A cette solution on ajoute 1,05 eq. de triéthylamine et le milieu est agité à température ambiante pendant une heure. La solution de sel de triéthylamine obtenue est alors ajoutée à une solution de chloroformiate d'alkyle ClCOOR' de préférence le chloroformiate d'isopropyle (1 eq.) dans le tétrahydrofuranne (10% P/V) refroidi à -20°C dans un bain alcool/carboglace. Après addition le milieu est laissé sous agitation au minimum trois heures.

Le 4-hydroxy-phényl-β-D-glucose (3 eq.) est parallèlement mis en solution dans un solvant basique tel que la pyridine anhydre (27g/100ml) et le milieu précédent éventuellement filtré est additionné à la solution de 4-hydroxy-phényl-β-D-glucose à température ambiante pendant au moins trois heures.

Après évaporation des solvants, le produit est :

- soit chromatographié sur colonne de gel de silice à l'aide d'un mélange de solvants à base de chlorure de méthylène et de méthanol,
- soit extrait à l'aide d'un mélange de solvants eau-acétate d'éthyle-heptane.

Selon ce procédé général, on a préparé les monoesters du 4-hydroxy-phényl-β-D-glucose suivants :

**EXEMPLE 1** : 4-hexanoyl-oxy-phényl-β-D-glucose

Rendement : 26%
F = 122-127°C
$[\alpha]^{20}_D$= -40° (C = 26mg/ml, éthanol)

| Analyse élémentaire : $C_{18}H_{26}O_8$ ; M = 370,4 | | |
|---|---|---|
| | C% | H% |
| Calc. | 58,37 | 7,08 |
| Tr. | 57,88 | 7,04 |

Les spectres de R.M.N. $^1H$ et $^{13}C$ (DMSO $D_6$) sont en accord avec la structure.

**EXEMPLE 2** : 4-décanoyl-oxy-phényl-β-D-glucose

Rendement : 36%
F = 157-159°C
$[\alpha]^{20}_D$= -23° (C = 44mg/ml, DMSO)

| Analyse élémentaire : $C_{22}H_{34}O_8$, 0,5 $H_2O$ ; M = 435,5 | | |
|---|---|---|
| | C% | H% |
| Calc. | 60,67 | 8,10 |
| Tr. | 60,78 | 7,89 |

Le spectre de R.M.N. du $^{13}C$ (méthanol $D_4$) est en accord avec la structure.

**EXEMPLE 3** : 4-dodécanoyl-oxy-phényl-β-D-glucose

Rendement : 25%
F = 114°C
$[\alpha]^{20}_{D}$= -25° (C = 34mg/ml, DMSO)

| Analyse élémentaire : $C_{24}H_{38}O_8$, 0,5 $H_2O$ ; M = 463,6 | | |
|---|---|---|
| | C% | H% |
| Calc. | 62,18 | 8,48 |
| Tr. | 61,95 | 8,31 |

Le spectre de R.M.N. du $^{13}C$ (DMSO $D_6$) est en accord avec la structure.

**EXEMPLE 4** : 4-hexadécanoyl-oxy-phényl-β-D-glucose

Rendement : 35%
F = 107°C
$[\alpha]^{20}_{D}$= -23° (C = 40mg/ml, DMSO)

| Analyse élémentaire : $C_{28}H_{46}O_8$ ; M = 510,7 | | |
|---|---|---|
| | C% | H% |
| Calc. | 65,86 | 9,08 |
| Tr. | 66,07 | 9,20 |

Le spectre de R.M.N. du $^{13}C$ (DMSO $D_6$) est en accord avec la structure.

**EXEMPLE 5** : 4-oléoyl-oxy-phényl-β-D-glucose

Rendement : 37%
F = 99°C
$[\alpha]^{20}_{D}$= -30° (C = 31mg/ml, éthanol)

| Analyse élémentaire : $C_{30}H_{48}O_8$ ; M = 536,7 | | |
|---|---|---|
| | C% | H% |
| Calc. | 67,14 | 9,01 |
| Tr. | 66,98 | 9,03 |

Le spectre de R.M.N. du $^{13}C$ (méthanol $D_4$) est en accord avec la structure.

**EXEMPLE 6** : 4-linoléoyl-oxy-phényl-β-D-glucose

Rendement : 30%
F = 95°C
$[\alpha]^{20}_{D}$= -27° (C = 50mg/ml, éthanol)

| Analyse élémentaire : $C_{30}H_{46}O_8$, 0,5 $H_2O$ ; M = 543,7 | | |
|---|---|---|
| | C% | H% |
| Calc. | 66,27 | 8,71 |
| Tr. | 66,51 | 8,39 |

Le spectre de R.M.N. du $^{13}C$ (DMSO $D_6$) est en accord avec la structure.

## EXEMPLES DE COMPOSITIONS COSMETIQUES

**EXEMPLE 1 :** Emulsion huile-dans-l'eau

. Alcool cétylique 4 g
. Tristéarate de Sorbitan 0,2 g
. Stéarate de polyéthylène glycol (40 OE) 2 g
. Palmito-stéarate de glycéryle 3 g
. Myristate de myristyle 7,5 g
. Polyisobutylène 6,2 g
. Silicone Volatile (Cyclométhicone) 5 g
. 4-hexanoyl-oxy-phényl-β-D-glucose 6,7 g
. Conservateurs 0,3 g
. Parfum 0,2 g
. Eau distillée qsp 100g

**EXEMPLE 2 :** Emulsion huile-dans-l'eau

. Huile de vaseline 5 g
. Huile de noyau d'abricot 6 g
. Beurre de karité (fraction liquide) 10 g
. Monostéarate de Sorbitan oxyéthyléné (20 OE) 1 g
. Stéarate de glycéryle-stéarate de polyéthylène glycol (100 OE) en mélange 50/50 1 g
. Acide stéarique 1,5 g
. Glycérol 3 g
. Filtres UVA-UVB 1,5 g
. Parfum 0,2 g
. Polymère carboxyvinylique (Carbopol 941) 0,2 g
. Triéthanolamine 0,5 g
. Conservateurs 0,2 g
. 4-hexanoyl-oxy-phényl-β-D-glucose 4 g
. Eau distillée qsp 100 g

**EXEMPLE 3 :** Préparation d'une dispersion vésiculaire sous forme de crème

On prépare tout d'abord une phase lipidique en procédant au mélange des ingrédients suivants:

- 4-hexadécanoyl-oxy-phényl-β-D-glucose 0,20g
- Cholestérol 0,75g
- Dicétylphosphate de sodium 0,10g
- Lipide non ionique de formule:

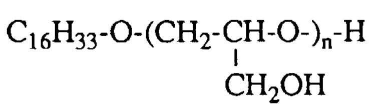

0,95g

où $\bar{n}$ = 3
- Dichlorométhane 10 ml
- Méthanol 10 ml

Lorsque la solubilisation est totale, les solvants sont évaporés à 40°C par paliers successifs depuis la pression ambiante jusqu'à environ 500 Pa à l'aide d'un évaporateur rotatif.

Au film de phase lipidique obtenu, on ajoute en 2 heures et à 70°C, 38g d'une solution de glucose 0,3M dans l'eau. Le mélange obtenu est agité à l'aide d'une secoueuse à bras oscillants commercialisée par la Société Prolabo sous la dénomination de "O.S.C.H.I 12". La dispersion obtenue peut être éventuellement homogénéisée.

**Revendications**

1. Monoesters du 4-hydroxy-phényl-β-D-glucose répondant à la formule générale suivante :

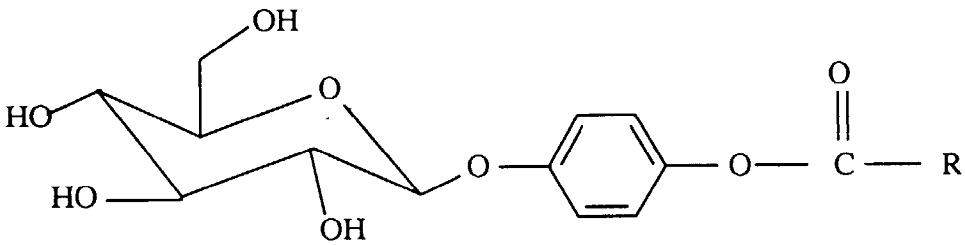

(I)

dans laquelle :
R représente un radical alkyle, linéaire ou ramifié ou alcényle ayant de 5 à 21 atomes de carbone ou un radical alcapolyényle ayant de 9 à 21 atomes de carbone ou R représente un mélange défini de tels radicaux alkyle, alcényle ou alcapolyényle.

2. Monoesters selon la revendication 1 caractérisés par le fait qu'ils sont choisis parmi :

4-hexanoyl-oxy-phényl-β-D-glucose,
4-décanoyl-oxy-phényl-β-D-glucose,
4-dodécanoyl-oxy-phényl-β-D-glucose,
4-hexadécanoyl-oxy-phényl-β-D-glucose,
4-oléoyl-oxy-phényl-β-D-glucose, et
4-linoléoyl-oxy-phényl-β-D-glucose.

3. Procédé de préparation des monoesters du 4-hydroxy-phényl-β-D-glucose selon les revendications 1 et 2, caractérisé par le fait qu'il consiste en un premier temps à former dans un solvant organique, un anhydride mixte de formule II :

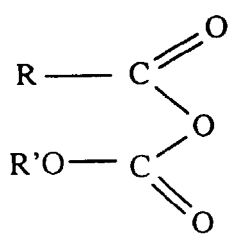

(II)

dans laquelle :
R représente un radical alkyle, linéaire ou ramifié ou alcényle, ayant de 5 à 21 atomes de carbone ou un radical alcapolyényle ayant de 9 à 21 atomes de carbone, ou R représente un mélange défini de tels radicaux alkyle, alcényle ou alcapolyényle et R' représente un radical alkyle, linéaire ou ramifié, ayant de 2 à 10 atomes de carbone, par réaction en présence d'une base, d'un acide R-COOH, R ayant la même signification que ci-dessus et d'un chloroformiate d'alkyle ClCOOR', R' ayant la même signification que ci-dessus, et dans un deuxième temps, à faire réagir ledit anhydride mixte de formule II en solution dans ledit solvant organique ou dans un mélange de solvants organiques, avec du 4-hydroxy-phényl-β-D-glucose, éventuellement en présence d'une base.

4. Procédé selon la revendication 3, caractérisé par le fait que le solvant organique est choisi parmi le tétrahydrofuranne, le N,N-diméthylformamide, la pyridine, la N-méthylpyrrolidone, le N,N-diméthylacétamide, le dichlorométhane ou un mélange de ces solvants.

5. Procédé selon les revendications 3 et 4, caractérisé par le fait que la base organique est choisie parmi la triéthylamine, la pyridine, la 4-diméthylaminopyridine, la tributylamine et la N-méthylmorpholine.

6. Procédé selon l'une quelconque des revendications 3 à 5, caractérisé par le fait que le 4-hydroxy-phényl-β-D-glucose est mis à réagir en solution dans de la pyridine, de la N-méthylpyrrolidone, du N,N-diméthylformamide amide ou du N,N-diméthylacétamide.

**7.** Procédé selon l'une quelconque des revendications 3 à 6, caractérisé par le fait que l'on fait réagir environ 3 équivalents de 4-hydroxy-phényl-β-D-glucose par rapport à l'acide mis à réagir.

**8.** Composition cosmétique, pharmaceutique, bucco-dentaire ou alimentaire caractérisée par le fait qu'elle contient dans un véhicule approprié au moins un monoester du 4-hydroxy-phényl-β-D-glucose tel que revendiqué selon l'une quelconque des revendications 1 et 2.

**9.** Composition selon la revendication 8, caractérisée par le fait que le monoester du 4-hydroxy-phényl-β-D-glucose est présent à une concentration comprise entre 0,01 et 30% et de préférence entre 0,1 et 15% en poids par rapport au poids total de la composition.

**10.** Utilisation des monoesters du 4-hydroxy-phényl-β-D-glucose tels que revendiqués selon les revendications 1 et 2 comme agents épaississants de phases huileuses et de solutions aqueuses.

**Patentansprüche**

**1.** Monoester der 4-Hydroxyphenyl-β-D-glucose gemäß folgender allgemeiner Formel:

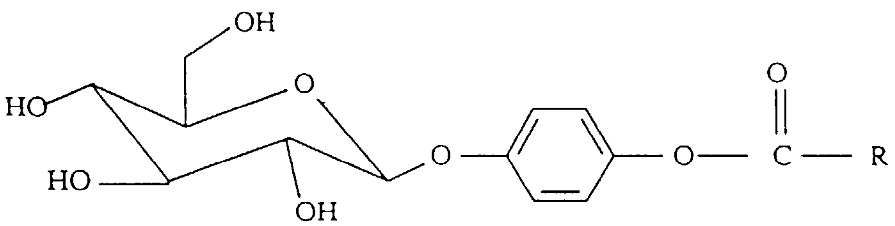

(I)

worin:
R einen gerad- oder verzweigtkettigen Alkylrest oder einen Alkenylrest mit jeweils 5 bis 21 Kohlenstoffatomen oder einen Alkapolyenylrest mit 9 bis 21 Kohlenstoffatomen bedeutet, oder R ein Gemisch darstellt, das durch derartige Alkyl-, Alkenyl- oder Alkapolyenylreste definiert ist.

**2.** Monoester nach Anspruch 1, dadurch gekennzeichnet, daß er unter

4-Hexanoyloxyphenyl-β-D-glucose,
4-Decanoyloxyphenyl-β-D-glucose,
4-Dodecanoyloxyphenyl-β-D-glucose,
4-Hexadecanoyloxyphenyl-β-D-glucose,
4-Oleoyloxyphenyl-β-D-glucose, sowie
4-Linoloyloxyphenyl-β-D-glucose

ausgewählt ist.

**3.** Verfahren zur Herstellung von Monoestern der 4-Hydroxyphenyl-β-D-glucose nach den Ansprüchen 1 und 2, dadurch gekennzeichnet, daß es darin besteht, in einem ersten Schritt in einem organischen Lösungsmittel ein gemischtes Anhydrid der Formel II zu bilden:

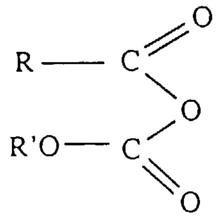

(II)

worin:

R einen gerad- oder verzweigtkettigen Alkylrest oder Alkenylrest mit 5 bis 21 Kohlenstoffatomen oder einen Alkapolyenylrest mit 9 bis 21 Kohlenstoffatomen darstellt oder R ein Gemisch bedeutet, das durch derartige Alkyl-, Alkenyloder Alkapolyenylreste definiert ist und R' einen geradoder verzweigtkettigen Alkylrest mit 2 bis 10 Kohlenstoffatomen bedeutet, und zwar

durch die Reaktion in Anwesenheit einer Base, einer Säure R-COOH, wobei R dieselbe Bedeutung aufweist wie oben angegeben, und eines Alkylchlorformiats ClCOOR', wobei R' dieselbe Bedeutung aufweist wie oben angegeben, und

in einem zweiten Schritt das gemischte Anhydrid gemäß der Formel II in Lösung in dem organischen Lösungsmittel oder in einem Gemisch aus organischen Lösungsmitteln mit der 4-Hydroxyphenyl-β-D-glucose, gegebenenfalls in Anwesenheit einer Base, zur Reaktion zu bringen.

**4.** Verfahren nach Anspruch 3, dadurch gekennzeichnet, daß das organische Lösungsmittel unter Tetrahydrofuran, N,N-Dimethylformamid, Pyridin, N-Methylpyrrolidon, N,N-Dimethylacetamid, Dichlormethan oder einem Gemisch dieser Lösungsmittel ausgewählt wird.

**5.** Verfahren nach den Ansprüchen 3 und 4, dadurch gekennzeichnet, daß die organische Base unter Triethylamin, Pyridin, 4-Dimethylaminopyridin, Tributylamin und N-Methylmorpholin ausgewählt wird.

**6.** Verfahren nach einem der Ansprüche 3 bis 5, dadurch gekennzeichnet, daß die 4-Hydroxyphenyl-β-D-glucose in Lösung in Pyridin, N-Methylpyrrolidon, N,N-Dimethylformamid oder N,N-Dimethylacetamid zur Reaktion gebracht wird.

**7.** Verfahren nach einem der Ansprüche 3 bis 6, dadurch gekennzeichnet, daß etwa drei Äquivalente der 4-Hydroxyphenyl-β-D-glucose in bezug auf die Säure zur Reaktion gebracht werden.

**8.** Kosmetische, pharmazeutische, bukkal-dentale oder Lebensmittelzubereitung, dadurch gekennzeichnet, daß sie in einem geeigneten Träger mindestens einen Monoester der 4-Hydroxyphenyl-β-D-glucose, wie er in einem der Ansprüche 1 oder 2 beansprucht ist, enthält.

**9.** Zubereitung nach Anspruch 8, dadurch gekennzeichnet, daß der Monoester der 4-Hydroxyphenyl-β-D-glucose in einer Konzentration zwischen 0,01 und 30 Gew.-%, vorzugsweise zwischen 0,1 und 15 Gew.-% in bezug auf das Gesamtgewicht der Zusammensetzung vorhanden ist.

**10.** Verwendung von Monoestern der 4-Hydroxyphenyl-β-D-glucose, wie sie in den Ansprüchen 1 und 2 beansprucht sind, als Verdickungsmittel von Ölphasen und wäßrigen Lösungen.

## Claims

**1.** Monoesters of 4-hydroxyphenyl-β-D-glucose corresponding to the following general formula:

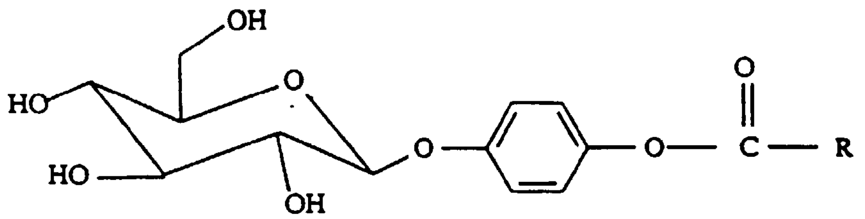

(I)

in which:
R represents a linear or branched alkyl radical or an alkenyl radical having from 5 to 21 carbon atoms or an alkapolyenyl radical having from 9 to 21 carbon atoms or R represents a defined mixture of such alkyl, alkenyl or alkapolyenyl radicals.

2. Monoesters according to Claim 1, characterized in that they are chosen from:

    4-hexanoyloxyphenyl-β-D-glucose,
    4-decanoyloxyphenyl-β-D-glucose,
    4-dodecanoyloxyphenyl-β-D-glucose,
    4-hexadecanoyloxyphenyl-β-D-glucose,
    4-oleoyloxyphenyl-β-D-glucose, and
    4-linoleoyloxyphenyl-β-D-glucose.

3. Process for the preparation of the monoesters of 4-hydroxyphenyl-β-D-glucose according to Claims 1 and 2, characterized in that it consists, in a first stage, in forming, in an organic solvent, a mixed anhydride of formula II:

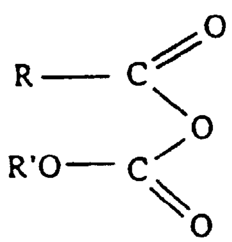

(II)

in which:
R represents a linear or branched alkyl radical or an alkenyl radical, having from 5 to 21 carbon atoms, or an alkapolyenyl radical having from 9 to 21 carbon atoms, or R represents a defined mixture of such alkyl, alkenyl or alkapolyenyl radicals and R' represents a linear or branched alkyl radical having from 2 to 10 carbon atoms, by reacting, in the presence of a base, an acid R-COOH, R having the same meaning as above, and an alkyl chloroformate C1COOR', R' having the same meaning as above, and in a second stage, in reacting the said mixed anhydride of formula II, in solution in the said organic solvent or in a mixture of organic solvents, with 4-hydroxyphenyl-β-D-glucose, optionally in the presence of a base.

4. Process according to Claim 3, characterized in that the organic solvent is chosen from tetrahydrofuran, N,N-dimethylformamide, pyridine, N-methylpyrrolidone, N,N-dimethylacetamide, dichloromethane or a mixture of these solvents.

5. Process according to Claims 3 and 4, characterized in that the organic base is chosen from triethylamine, pyridine, 4-dimethylaminopyridine, tributylamine and N-methylmorpholine.

6. Process according to any one of Claims 3 to 5, characterized in that 4-hydroxyphenyl-β-D-glucose is reacted in solution in pyridine, N-methylpyrrolidone, N,N-dimethylformamide or N,N-dimethylacetamide.

7. Process according to any one of Claims 3 to 6, characterized in that about 3 equivalents of 4-hydroxyphenyl-β-D-glucose are reacted relative to the acid which is reacted.

8. Cosmetic, pharmaceutical, dentibuccal or food composition, characterized in that it contains, in an appropriate vehicle, at least one monoester of 4-hydroxyphenyl-β-D-glucose as claimed in either of Claims 1 and 2.

9. Composition according to Claim 8, characterized in that the monoester of 4-hydroxyphenyl-β-D-glucose is present at a concentration of between 0.01 and 30%, and preferably between 0.1 and 15% by weight relative to the total weight of the composition.

10. Use of the monoesters of 4-hydroxyphenyl-β-D-glucose as claimed in Claims 1 and 2, as thickening agents for oily phases and aqueous solutions.